# EUROPEAN PATENT APPLICATION

(11) **EP 3 845 123 A1**
(43) Date of publication of application: **07.07.2021**
(21) Application number: 19854018.9
(22) Date of filing: 27.08.2019
(51) Int. Cl.: A61B 5/022, A61B 5/00, B60N 2/90

(54) **BLOOD PRESSURE MEASUREMENT DEVICE, VEHICLE DEVICE, AND BLOOD PRESSURE MEASUREMENT PROGRAM**

(30) Priority: 27.08.2018 JP 2018158788
(71) Applicant: Equos Research Co., Ltd., Tokyo 101-0021 (JP); National University Corporation Iwate University, Morioka-shi, Iwate 020-8550 (JP)
(72) Inventor: SATO, Atsushi, Tokyo 101-0021 (JP); HONMA, Naoki, Morioka-shi, Iwate 020-8550 (JP); KOBAYASHI, Koichiro, Morioka-shi, Iwate 020-8550 (JP); IWAI, Morio, Morioka-shi, Iwate 020-8550 (JP)
(74) Representative: TBK
(86) International application number: PCT/JP2019/033454
(87) International publication number: WO 2020/045400

(57) **Abstract**

The purpose of the present invention is to measure the blood pressure of a living body in a noncontact and non-invasive manner. An experimental device 1 irradiates a subject 7 with microwaves from a transmission antenna 23 and a reception antenna 25 receives the reflected waves. Since the phase of the reflected wave varies with micro-movements of the body surface caused by the heartbeat, a pulse wave can be detected from the difference in phase between the emitted wave and the reflected wave. Additionally, the experimental device 1 continuously detects the blood pressure of the subject 7 from a blood pressure sensor 5 mounted on the subject 7 while continuously detecting the pulse wave. A frequency spectrum of the pulse wave of the subject 7 is generated by Fourier transformation of the pulse wave. A comparison of the spectrum and the blood pressure of the subject 7 detected by the blood pressure sensor 5 reveals a statistically significant positive correlation of the systolic blood pressure with an eighth harmonic wave from among frequency components of the pulse wave. On the basis of the experimental result, a device can be realized which detects a pulse wave of a subject in a noncontact and non-invasive manner using a microwave, and which measures the systolic blood pressure using the correlation therebetween.

## Description

### TECHNICAL FIELD

The present invention relates to a blood pressure measurement device, a vehicle device, and a blood pressure measurement program, and relates to those for measuring a blood pressure from pulse waves, for example.

### BACKGROUND ART

In recent years, health consciousness has been increasing in combination with declining birthrate and aging population or the like, and blood pressure measurement in daily life has become more important from a viewpoint of disease prevention and early detection.

Blood pressure measurement methods include, for example: a measurement method in a non-invasive manner for directly or indirectly measuring a pulse wave velocity, pulse wave acceleration, or the like to be converted into a blood pressure by mounting a special purpose device on an arm or a fingertip; a measurement method in an invasive manner realized by directly inserting a balloon or the like in a blood vessel; and the like.

In particular the non-invasive manner has been actively studied as described in the "Guidelines for non-invasive vascular function test" by the Japanese Circulation Society since the non-invasive manner has a smaller load on a human body is than that of the invasive manner.

By the way, even in the blood pressure measurement in the non-invasive manner, it is necessary to mount some kind of device on a subject (measured person), such as mounting a cuff on an arm or a fingertip. Therefore, there was a problem of imposing physical and psychological burden upon the subject. For example, depending on the device, it is necessary to apply a pressure by the cuff or the like, so that the blood pressure may be fluctuated due to unpleasant feelings, and as a result, some error may occur in a measured value. The white robe effect is well-known as a psychogenic phenomenon.

Moreover, when monitoring a blood pressure of a driver during driving a vehicle, it is not realistic to mount a device on the driver every time.

Therefore, a blood pressure measurement method in a noncontact manner has been required.

However, there is a high demand for such a non-invasive manner and noncontact manner vital measurement since it has a small load provided to the subject, and therefore there has been designed a technique for measuring a breathing rate and a pulse rate of the subject using a microwave, a photo sensor, and a camera. However, the technique for measuring blood pressure in a noncontact manner to a living body has not even established a methodology such as what and how to measure the blood pressure in the noncontact manner. Therefore, due to a technical difficulty thereof, a method that can be used in daily life (in particular can be used during driving a vehicle) has not yet been realized.

For example, a technique disclosed in Patent Literature 1 is a technique which measures a propagation velocity of a pulse wave by irradiating two points of a living body with a microwave and then measures a blood pressure in a noncontact manner on the basis of a theory that there is a correlation between a propagation velocity of the pulse wave and the blood pressure.

This technology has limitations for use in daily life, such as irradiating two predetermined points of the arm portion or the like with the microwave, for example, while the subject is in a resting state lying on a bed, and therefore it is difficult to be used in particular in vehicles.

### CITATION LIST

### PATENT LITERATURE

Patent Literature 1: Japanese Patent Application Publication No. 2014-230671

### DISCLOSURE OF INVENTION

### PROBLEM TO BE SOLVED BY THE INVENTION

The object of the present invention is to measure a blood pressure of a living body in a noncontact and non-invasive manner.

### SUMMARY OF THE INVENTION(S)

(1) The invention described in claim 1 provides a blood pressure measurement device comprising: a pulse wave obtaining means configured to obtain a pulse wave from a living body; a frequency component obtaining means configured to obtain a frequency component of the obtained pulse wave; and a blood pressure measurement means configured to measure a blood pressure of the living body using a frequency component in a band of a predetermined range at a side of a high-frequency range of a fundamental frequency due to the pulse wave, among the obtained frequency components.
(2) The invention described in claim 2 provides the blood pressure measurement device according to claim 1, wherein the blood pressure measurement means adopts a band in which a correlation between the frequency component of the pulse wave and the blood pressure is equal to or greater than a predetermined level as the band of the predetermined range.
(3) The invention described in claim 3 provides the blood pressure measurement device according to claim 1 or 2, wherein the blood pressure measurement means measures the blood pressure using a component of a harmonic of the fundamental frequency in the band of the predetermined range.
(4) The invention described in claim 4 provides the blood pressure measurement device according to claim 1, 2, or 3, wherein the pulse wave obtaining means obtains the pulse wave from a body surface of the living body in a noncontact manner.
(5) The invention described in claim 5 provides the blood pressure measurement device according to claim 4, wherein the pulse wave obtaining means irradiates the living body with an electromagnetic wave having a predetermined frequency and obtains the pulse wave using a reflected wave of the electromagnetic wave.
(6) The invention described in claim 6 provides the blood pressure measurement device according to claim 5, further comprising an informing means configured to inform a content according to a change in the measured blood pressure.
(7) The invention described in claim 7 provides a vehicle device comprising the blood pressure measurement device according to claim 6 configured to measure a blood pressure of a driver in a vehicle.
(8) The invention described in claim 8 provides the vehicle device according to claim 7, wherein an antenna for irradiating the driver with the electromagnetic wave and an antenna for receiving the reflected wave of the electromagnetic wave are provided on a backrest portion of a driver's seat.
(9) The invention described in claim 9 provides a blood pressure measurement program for causing a computer to realize: a pulse wave obtaining function for obtaining a pulse wave from a living body; a frequency component obtaining function for obtaining a frequency component of the obtained pulse wave; and a blood pressure measurement function for measuring a blood pressure of the living body using a frequency component in a band of a predetermined range at a side of a high-frequency range of a fundamental frequency due to the pulse wave, among the obtained frequency components.

### EFFECT OF THE INVENTION(S)

According to the present invention, the blood pressure of the living body can be measured in the noncontact and non-invasive manner by using a frequency component of the pulse wave.

### BRIEF DESCRIPTION OF DRAWINGS

FIG. 1 is a diagram for describing a configuration of an experimental device.
FIG. 2 is a diagram illustrating equations.
FIG. 3 is a graphic chart illustrating a waveform of a pulse wave.
FIG. 4 is a graphic chart illustrating a frequency spectrum of the pulse wave.
FIG. 5 is a scatter chart plotting a correspondence between an electric power value of an eighth harmonic wave and a systolic blood pressure.
FIG. 6 is a graphic chart illustrating a correlation coefficient between a reflected power value and the systolic blood pressure.
FIG. 7 is a diagram illustrating a configuration of a blood pressure measurement device.
Fig. 8 is a flow chart for describing a procedure of blood pressure detection processing.

### BEST MODE(S) FOR CARRYING OUT THE INVENTION

### (1) Outline of Embodiments

An experimental device 1 irradiates a subject (measured person) 7 with a microwave from a transmission antenna 23 and receives a reflected wave with a reception antenna 25. Since a phase of the reflected wave varies with micro-movements of a body surface caused by a heartbeat, a pulse wave can be detected from a phase difference between the emitted wave and the reflected wave.

Additionally, the experimental device 1 continuously detects a blood pressure of the subject 7 from a blood pressure sensor 5 mounted on the subject 7 while continuously detecting the pulse wave.

The inventors of the present application generated a frequency spectrum of the pulse wave of the subject 7 by Fourier transformation of the pulse wave and compared the generated frequency spectrum and the blood pressure of the subject 7 detected by the blood pressure sensor 5, and thereby found that a frequency component in a band of a predetermined range at a side of a high-frequency range of a fundamental frequency from among frequency components of the pulse wave shows a statistically significant positive correlation with a systolic blood pressure. In particular, the inventors found that an eighth harmonic wave shows the statistically significant positive correlation with the systolic blood pressure.

On the basis of the experimental result, a device can be realized which detects a pulse wave of a subject in a noncontact and non-invasive manner using a microwave, and which measures (estimates) the systolic blood pressure using the correlation therebetween. This allows for continuous measurement of the blood pressure without discomfort or hassle.

Moreover, in the present embodiment, since the blood pressure is measured by frequency analysis the frequency component of the pulse wave instead of the propagation velocity of the pulse wave, the number of the microwave circuits 2 for irradiating it with microwave can be reduced to one instead of two.

### (2) Details of the embodiments

First, the experimental device will now be described.

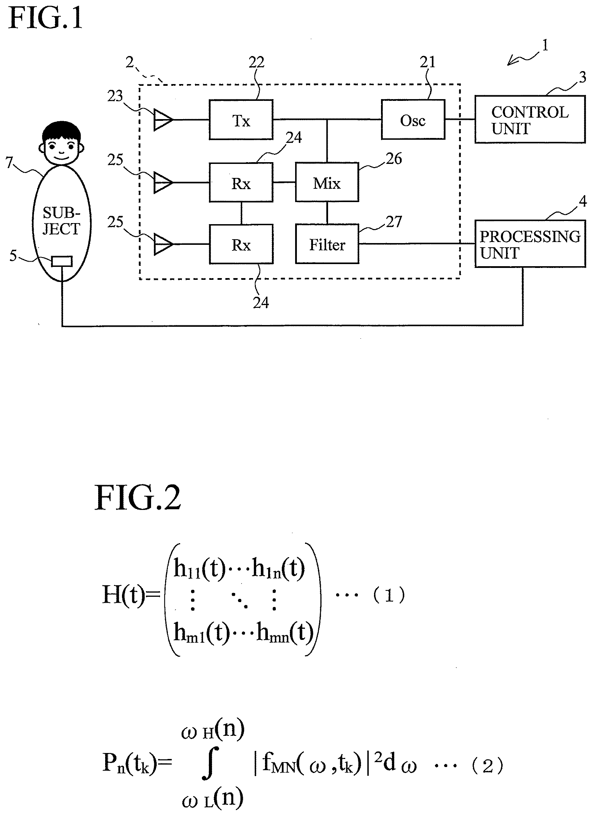

FIG. 1 is a diagram for describing a configuration of the experimental device 1 for investigating a correlation between a pulse wave and a blood pressure.

The experimental device 1 is configured of a microwave circuit 2, a control unit 3, a processing unit 4, a blood pressure sensor 5, and the like.

The microwave circuit 2 is configured of an oscillator 21, a transmitter 22, a transmission antenna 23, a receiver 24, a reception antenna 25, a mixer 26, a filter 27, and the like.

Moreover, although not illustrated, a chair for the subject 7 to sit down is installed in a direction of microwave irradiation by the microwave circuit 2.

In addition, as will be described later, in the configuration of the experimental device 1, a portion except for the blood pressure sensor 5 is incorporated in a blood pressure measurement device 40 for monitoring a blood pressure of a driver in a vehicle, and is used for measuring (estimating) the blood pressure of a measured person (e.g., a driver, a hospital patient, or the like).

The oscillator 21 includes a device for oscillating the microwave, and generates the microwaves having a predetermined frequency as a continuous wave and outputs the generated microwaves to the transmitter 22, and also outputs a part of the microwaves to the mixer 26 as a reference wave.

In the embodiments, a microwave of 5 [GHz] generated by the oscillator 21 is used as an example, as electromagnetic waves for detecting the pulse wave.

The transmitter 22 transmits the microwave generated by the oscillator 21 from the transmission antenna 23 to space.

A sixteenth-element patch array antenna is used for the transmission antenna 23. Although there are sixteenth sets of the transmitter 22 and the transmission antenna 23, merely one set is illustrated in FIG. 1.

The receiver 24 receives a reflected wave, in which the irradiated wave (emitted wave) by the transmitter 22 is reflected back from the target (subject 7), with the reception antenna 25, and transmits this reflected wave to the mixer 26.

A four-element patch array antenna is used for the reception antenna 25. Although there are four sets of the reception antenna 25 and the receiver 24, merely two sets are illustrated in FIG. 1.

As described above, the experimental device 1 has a Multiple Input Multiple Output (MIMO) including 16 transmission elements and four reception elements. Moreover, an interval between these elements is 0.5 wavelength.

With such a configuration, directivity of the microwave can be sharpened, and also a direction of the microwave can be finely adjusted to irradiate, with the microwave, a region where the pulse wave can be well detected (around the heart of the subject 7, in accordance with the inventor's experiment).

The mixer 26 mixes the reflected wave received by the receiver 24 with the reference wave by the oscillator 21, thereby generating beats (beat, mixed wave) to be output to the filter 27.

The filter 27 is a filter configured to pass only a band required for measurement of the pulse wave among the beat waves output by the mixer 26.

The body surface of the subject 7 is finely vibrated by activity of the cardiopulmonary, and fine fluctuation of a reflecting surface (body surface) due to this vibration causes a phase change (microwave Doppler) in the reflected wave.

Then, when the irradiated wave of a fixed frequency as a reference wave is mixed with the reflected wave, a beat based on a phase difference is generated, and the pulse wave can be detected and reproduced by using this beat.

In this manner, the experimental device 1 can measure the vibration of the body surface in the noncontact and non-invasive manner.

Even if the subject 7 wears clothes made of cotton or synthetic fibers, the microwave passes through such clothes, and therefore the subject 7 can remain clothed. As described above, since the above-mentioned method using the microwave can be used in the noncontact manner and clothed state, it is suitable for measuring the blood pressure in daily life, such as at the time of driving a vehicle.

As described above, the experimental device 1 includes the pulse wave obtaining means configured to irradiate the living body (subject 7) with the electromagnetic waves having the predetermined frequency and to obtain the pulse wave from the living body using the reflected wave thereof, and thereby the pulse wave can be obtained from the body surface of the aforementioned living body in the noncontact manner.

The blood pressure sensor 5 is configured by using a cuff mounted on the right index finger of the subject 7, and thereby enabling to observe a time variation of the blood pressure with a sphygmomanometer from fluctuation of a pressure value due to a blood flow.

The experimental device 1 uses a continuous sphygmomanometer (Finometer Musical Instrument Digital Interface (MIDI)) in order to measuring an actual blood pressure value of the subject 7 simultaneously with the detection of the pulse wave by the microwave circuit 2.

The control unit 3 is a control unit configured to control a drive of the oscillator 21.

The processing unit 4 is configured of an oscilloscope configured to display a pulse wave in accordance with a signal output from the microwave circuit 2, a spectrum analyzer configured to convert the pulse wave into a frequency spectrum by Fourier transform by Fast Fourier transform (FFT), a sphygmomanometer configured to measure the blood pressure of the subject 7 with the blood pressure sensor 5, a computer configured to analyze information obtained from these components, and the like.

As described above, the experimental device 1 can continuously obtain the pulse wave of the subject 7 by using the microwave in the noncontact manner, and in parallel therewith continuously can detect the blood pressure of the subject 7 by a blood pressure sensor 5.

In this experiment, the correlation between the pulse wave and the systolic blood pressure (SBP) is investigated by comparing the pulse wave detected by suing the microwave and the blood pressure detected by the blood pressure sensor 5.

Next, a method for evaluating the correlation characteristic between the pulse wave and the systolic blood pressure will be described.

In general, when there are n transmitting elements and m receiving elements, a variable MIMO channel matrix H (t) is represented as a matrix of m rows and n columns as shown in the equation (1) in FIG. 2.

In this experiment, a propagation channel having the highest time average power is selected to conduct the experiment from among the combinations of the receiving elements (reception antennas 25) and the transmitting elements (transmission antennas 23).

If the combination at this time has m receiving elements and n transmitting elements, respectively, a propagation channel coefficient of the aforementioned propagation channel is expressed as h <<mn>> (t).

Although mn is expressed as a subscript of h in the equation (1) shown in FIG. 2, the subscript portion shall be expressed by being enclosed in angle brackets, such as "<<mn>>" in this description. Hereinafter, the same description will be made.

Moreover, this experiment observes a correspondence between an electric power value for each frequency component of the pulse wave (electric power value of the frequency component caused by biological information) and an actual systolic blood pressure value, in a certain time interval Δt.

As described above, the experimental device 1 includes the frequency component obtaining means configured to obtain the frequency component of the pulse wave as an electric power value. Note that the electric power value may be an absolute value or a relative value with respect to some reference value.

A frequency response obtained by Fourier-transforming the kth propagation channel separated by arbitrary time interval Δt is expressed as f <<mn>> (ω, t <<k>>), where ω is an angular frequency and tk is time of kth interval.

n is an order of a harmonic on the basis of the fundamental frequency of the heart rate of the subject 7, a lower limit of the frequency component corresponding to each order is ω <<L>> (n), and an upper limit corresponding thereto is ω <<H>> (n).

For example, since the heart of the subject 7 beats approximately 60 times per minute, the fundamental frequency is approximately 1 [Hz] (generally approximately 0.7 to 1.5 [Hz]), and the frequencies of secondary, tertiary, ... respectively are approximately 2 [Hz], 3 [Hz],

Electric power P <<n>> (t <<k>>) of the nth harmonic wave component is expressed by the equation (2) shown in FIG. 2.

In the embodiments, the electric power Pn (tk) of the fundamental component and the harmonic component of the pulse wave waveform of the subject 7 is obtained by Fourier transform using the FFT, and the average value of the actual systolic blood pressure corresponding to the time interval for which the Pn (tk) is obtained is obtained by the blood pressure sensor 5, thereby evaluating the correlation characteristics therebetween.

Next, implementation of the experiment will now be described.

The present experiment is an experiment for investigating a possibility of the noncontact manner blood pressure measurement using the microwave by comparing, with the actual systolic blood pressure, a reflection response of the microwave with which the living body is irradiated, and evaluating the correlation characteristic therebetween.

In a present experiment, channel measuring time is set to 210 seconds, and a sampling frequency (MIMO channel acquisition speed) is set to 200 [Hz].

The time interval Δt for performing the Fourier analysis is set to 5.12 seconds in terms of time and the number of samples is set to 1024 in consideration of the frequency resolution, and a humming window is used for the Fourier transform.

The present experiment is conducted with the cooperation of seven healthy men in their twenties, and respective actual continuously blood pressure values of the respective subjects 7 are simultaneously measured using the respective blood pressure sensors 5.

In order to reduce influence of body movement of the subjects 7, the subjects 7 are seated at a distance of 30 cm in the front direction of the antenna in a resting state.

In the present experiment, in order to prompt an arbitrary blood pressure changes while keeping the subjects 7 in a resting state and to verify changes of the reflected wave due to the blood pressure value, the subjects 7 wear goggles used for Virtual Reality (VR) and is given a video stimulus after the elapse of 60 seconds after the start of the measurement.

Next, an experimental result will now be described.

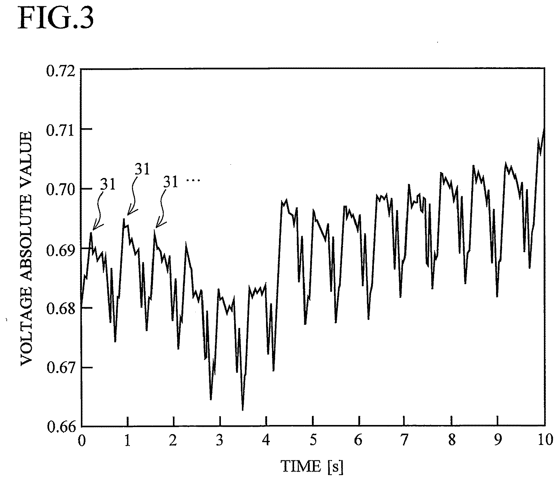

FIG. 3 is a graphic chart illustrating a waveform of a pulse wave of a subject 7 detected form the microwaves.

The vertical axis shows an absolute value of a detected voltage, and the horizontal axis shows a time period in seconds. As illustrated in the chart, pulse waves 31, 31, 31, ... due to heartbeat have appeared approximately once per second.

As illustrated in the chart, in the pulse waves 31, fine features of the waveform are also well detected, and extraction of features of the pulse waves 31 by means of frequency analysis can be expected.

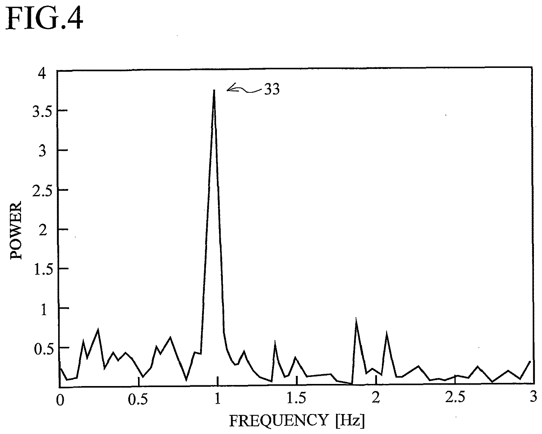

FIG. 4 is a graphic chart illustrating a frequency spectrum of the pulse wave.

This waveform is obtained by transforming the pulse wave 31 into a frequency region by the Fourier transform, and shows a level (power) of each frequency component composing the pulse wave.

The vertical axis shows a level of the frequency component as power (voltage value), and the horizontal axis shows the frequency.

As is clear from the graphic chart, the component 33 of approximately 1 [Hz] due to the heartbeat, which is a fundamental wave of the pulse wave, is contained most.

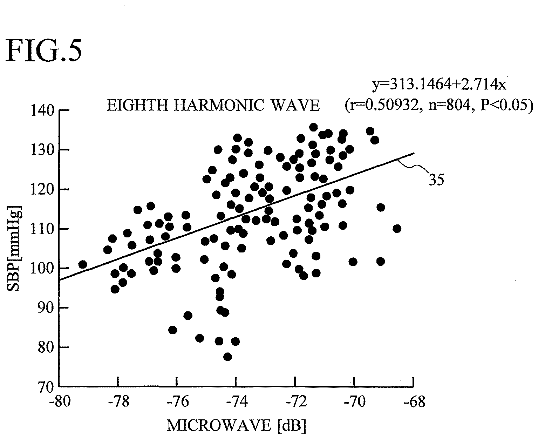

FIG. 5 is a scatter chart plotting a correspondence between electric power values (horizontal axis) of an eighth harmonic wave of the reflected waves and systolic blood pressures (SBP: vertical axis) in all subjects.

The electric power value of the eighth harmonic wave is obtained by Fourier-transforming the pulse wave 31, and the systolic blood pressure is measured using the blood pressure sensor 5.

In the present experiment, when a similar scatter chart is created from the fundamental wave to the twentieth harmonic wave and the correlation coefficient is obtained, since the eighth harmonic wave has the highest correlation coefficient, the scatter chart of the eighth harmonic wave is cited herein.

The straight line 35 illustrated in FIG. 5 represents a regression line obtained by the least squares method.

As illustrated in FIG. 5, the regression line is y = 313.1464 + 2.714 x, the correlation coefficient r is 0.50932, the number of samples n is 804, and the p-value is less than 0.05 with respect to the confidence interval of 95%, and therefore a significant positive correlation tendency is observed.

In this study, the correlation coefficient is calculated using Pearson's product moment correlation, and the p-value of less than 0.05 is considered statistically significant.

By the way, as a result of similarly analyzing the fundamental wave, the regression line is y = 148.7285 + 0.61697x, the correlation coefficient r is 0.32265, and the p-value is less than 0.05 with respect to the confidence interval of 95%, and therefore a significant positive correlation tendency is observed.

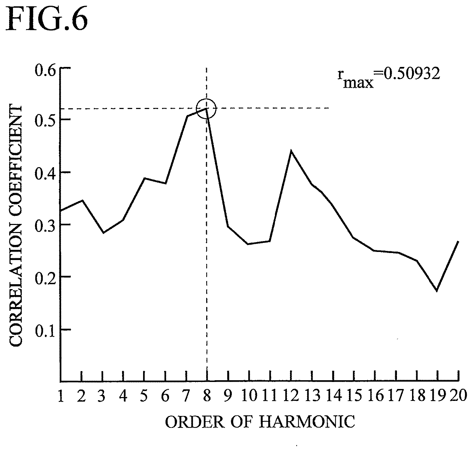

FIG. 6 is a graphic chart illustrating a correlation coefficient between the reflected power value and the systolic blood pressure for each harmonic wave from the fundamental wave to the twentieth harmonic wave.

As is clear from this chart, the correspondence between the eighth harmonic wave component of the reception power due to the pulse wave and the systolic blood pressure has the highest correlation coefficient, and a value thereof is 0.50932 as mentioned above.

Accordingly, it is considered that: it is preferable to use the eighth harmonic wave component of the pulse wave when measuring the systolic blood pressure on the basis of the pulse wave; and the systolic blood pressure can be measured on the basis of the level of the aforementioned component using the above-mentioned correlation.

In addition, in the present experiment, the correlation coefficient is measured for each harmonic frequency, and it is found out that the eighth harmonic wave frequency has the highest correlation. However, if the correspondence relationship between the correlation coefficient and the frequency is investigated in more detail, there is a possibility that a position slightly deviated from the frequency of the eighth harmonic wave may be the highest.

However, it is considered from the shape of the graphic chart that the most preferable frequency component used for the blood pressure measurement exists around the frequency of the eighth harmonic wave. There is also observed a peak near the twelfth harmonic wave, although not as large as the eighth harmonic wave.

Moreover, in the present experiment, the microwave of 5 [GHz] is used, but when microwaves of other frequencies are used, it is also considered that the correlation coefficient becomes higher at harmonic frequencies of other orders.

Therefore, it is considered that the frequency band preferable to the blood pressure measurement is a band in which the correlation coefficient is equal to or greater than a predetermined value between (n-1)th harmonic wave and (n+1)th harmonic wave centering on nth harmonic wave, and more preferably is near the nth harmonic wave in the aforementioned band or the frequency of the nth harmonic wave.

When the microwave frequency is 5 [GHz], the correlation coefficient is the highest near the eighth harmonic wave, i.e., n = 8. The band where the correlation coefficient between the frequency of the seventh harmonic wave and the frequency of the ninth harmonic wave is equal to or higher than 0.3, which is considered to have a correlation coefficient, or is equal to or higher than 0.4, which is considered to have a considerable correlation, and preferably is equal to or higher than 0.5, is a suitable band for the blood pressure measurement.

The blood pressure measurement device 40, which will be described later, measures the systolic blood pressure of the subject by comparing the level of eighth harmonic wave among the frequencies included in this band with the correlation illustrated in FIG. 5.

As described above, the blood pressure measurement device 40 includes the blood pressure measurement means configured to measure the blood pressure of the living body using the frequency component in the band of the predetermined range at the side of the high-frequency range of the fundamental frequency due to the pulse wave, among the frequency components. The blood pressure measurement means adopts the band in which the correlation between the frequency component of the pulse wave and the blood pressure is equal to or greater than a predetermined level as the band in the predetermined range used for the blood pressure measurement.

Moreover, the aforementioned blood pressure measurement means measures the blood pressure using the component of harmonics of the fundamental frequency in the band of the aforementioned predetermined range (the component of the eighth harmonic wave among the aforementioned bands in an example of the blood pressure measurement device 40).

In summary, in the present experiment, the living body is irradiated with the microwave and the correlation characteristics are evaluated from the correspondence between the microwave Doppler response and the actual blood pressure (in particular, the systolic blood pressure). The experimental result proved that there is a positive correlation relationship between the electric power value of the reflected wave and the systolic blood pressure value. Moreover, when the heart rate of the subject 7 is used as the fundamental frequency, a higher positive correlation is observed in the harmonic component thereof.

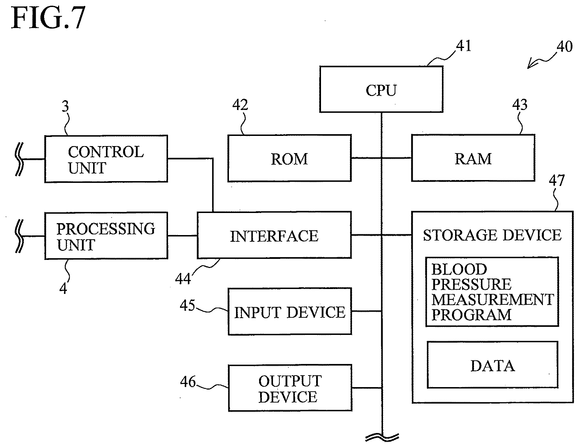

FIG. 7 is a diagram illustrating a configuration of a blood pressure measurement device 40 created using the above-mentioned experimental result.

The blood pressure measurement device 40 illustrated in FIG. 7 is miniaturized using a dedicated IC chip and the like and is mounted on a vehicle, for example, and will now be described as an example of a case of measuring and monitoring the blood pressure of a driver.

In addition, the blood pressure measurement device 40 can also be used for measuring the blood pressure on a daily basis at home, or measuring the blood pressure of patients at medical institutions.

As described above, a vehicle device of the aforementioned vehicle includes the blood pressure measurement device 40 configured to measure the blood pressure of the driver in the vehicle.

The blood pressure measurement device 40 is configured to using a Central Processing Unit (CPU) 41, a Read Only Memory (ROM) 42, a Random Access Memory (RAM) 43, an interface 44, an input device 45, an output device 46, a storage device 47, a control unit 3, a processing unit 4, and a microwave circuit 2 which is not illustrated.

The CPU 41 controls each part of the blood pressure measurement device 40 in accordance with a program stored by the ROM 42 or the storage device 47, and executes various arithmetic operations.

In the embodiments, the CPU 41 cooperatively works with the control unit 3 and/or the processing unit 4 to obtain the pulse wave of the subject (driver) for blood pressure measurement, and executes processing for measuring the blood pressure of the subject on the basis of the obtained pulse wave, and the like.

The ROM 42 is a read-only memory storing a basic program, parameters, and the like for operating the blood pressure measurement device 40.

The RAM 43 is a memory that can be read and written, and provides a working memory at the time of the CPU 41 executing various information processing by temporarily storing a blood pressure measurement program and data stored in the storage device 47, for example.

The interface 44 is an interface for connecting the control unit 3 and the processing unit 4 to the CPU 41. The CPU 41 can operate the microwave circuit 2 and can receive a level of the eighth harmonic wave from the processing unit 4, by controlling the control unit 3 through the interface 44.

The input device 45 includes an input device, such as a touch panel, a keyboard, and/or a mouse, and receives operations from a user of the blood pressure measurement device 40, for example.

The output device 46 includes an output device, such as a display, a loudspeaker, and/or a printer, displays an operation screen of the blood pressure measurement device 40 on the display, and informs a measured value of blood pressure, an alarm due to change of the blood pressure, and the like to the subject using the display, the loudspeaker, and/or the printer, for example.

Even if a heartbeat of a driver is normal, a drop in blood pressure may occur that requires attention. In such a case, the blood pressure measurement device 40 detects the change in the blood pressure as soon as possible, and changes information contents, such as a notification of the blood pressure, advice on rest, an emergency call, ..., and the like, in accordance with importance thereof, for example. Specifically, if it is determined that the blood pressure is continuously dropping, rather than dropping due to fluctuations, it determines that there is a possibility of reaching shock disease, and the blood pressure drop is informed based on a width and a rate of the drop.

Moreover, if the blood pressure rises above a predetermined value, the subject is considered to be tense and can be advised to take a break as well.

As described above, the blood pressure measurement device 40 includes the informing means configured to inform the content according to the change in the measured blood pressure.

The storage device 47 includes large-capacity media, such as a semiconductor memory device and/or a hard disk, for example. The storage device 47 stores the blood pressure measurement program that enables the CPU 41 to perform a function for obtaining the level of the eighth harmonic wave from the processing unit 4 and calculating the blood pressure value on the basis of correlation between the blood pressure and the obtained the level; other programs; data of past measurements value; and the like.

The control unit 3 controls a drive of the microwave circuit 2, which is not illustrated, in accordance with a signal from the CPU 41.

The processing unit 4 includes a function for detecting the pulse wave from the reflected wave of the microwave, and a function for Fourier-transforming the detected pulse wave and outputting the level of the eighth harmonic wave to the CPU 41.

Both the control unit 3 and the processing unit 4 are formed into an IC chip so as to be limited to the required functions, and are miniaturized enough to be installed in a vehicle.

The transmission antenna 23 and the reception antenna 25 composing the microwave circuit 2, which is not illustrated, are embedded into a backrest portion of a driver's seat. The transmission antenna 23 irradiates a back portion near the driver's heart with the microwave, and the reception antenna 25 receives a reflected wave modulated by the pulse wave. However, the transmission antenna 23 and the reception antenna 25 may be disposed on a dashboard, a steering wheel, an upper part of a windshield, or the like so as to irradiate near the heart with the microwave from in front of the driver.

Accordingly, the blood pressure measurement device 40 can detect the pulse wave of the driver in the noncontact manner and clothed state.

As described above, the blood pressure measurement device 40 includes the antenna for irradiating the driver with the electromagnetic wave and the antenna for receiving the reflected wave thereof, on a backrest portion of the driver's seat.

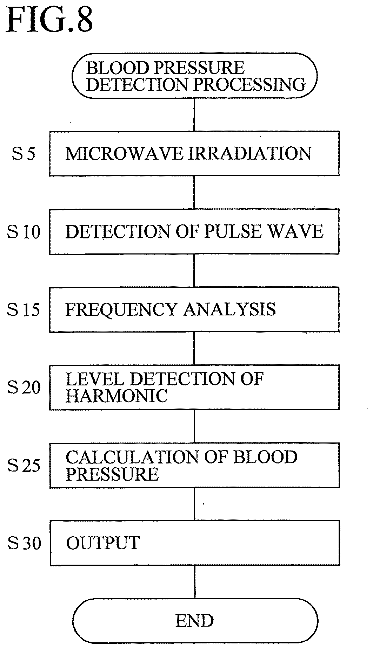

FIG. 8 is a flow chart for describing a procedure of blood pressure detection processing for detecting the systolic blood pressure executed by the blood pressure measurement device 40.

The following processing is processing executed by the CPU 41 in cooperation with the control unit 3 and the processing unit 4 in accordance with the blood pressure measurement program.

First, the CPU 41 irradiates the subject (a driver of a vehicle, a domestic user, a patient of a hospital, or the like) with the microwave from the microwave circuit 2 by driving the control unit 3 (Step 5).

In response thereto, the processing unit 4 detects the pulse wave from the reflected wave reflected from the subject (Step 10), and further frequency analysis the pulse wave (Step 15).

Furthermore, the processing unit 4 detects the level value of the eighth harmonic wave and transmits the detected level value to the CPU 41 (Step 20).

In response thereto, the CPU 41 receives the level value from the processing unit 4 and stores the received level value to the RAM 43.

Next, the CPU 41 performs calculation by substituting the aforementioned level value into a predetermined calculation formula using the correlation between the level value of the eighth harmonic wave stored in the RAM 43 and the systolic blood pressure, and stores the measured value of the systolic blood pressure calculated by the aforementioned calculation in the RAM 43 as a measured value (Step 25).

Then, the CPU 41 outputs the blood pressure stored in the RAM 43 to the output device 46 (Step 30).

In this manner, the blood pressure measurement device 40 can measure the systolic blood pressure of the subject in the noncontact and non-invasive manner and in the clothed state, and can detect vital signs by a unique algorithm.

As described above, the blood pressure measurement device 40 detects the pulse wave from the reflection response of the microwave with which the living body is irradiated, and executes the frequency component decomposition of the pulse wave, and thereby can measure the systolic blood pressure using the high-order component electric power of the fundamental frequency of the pulse wave (approximately 0.7 to 1.5 Hz).

This makes it possible to easily measure daily vitals in the noncontact and non-invasive manner and in the clothed state, and it is also an effective means of detecting signs of illness, in addition to monitoring the health status.

Although an example of the embodiments has been described above, various working examples and modified examples can also be realized.

For example, although the MIMO is used for the blood pressure measurement device 40, it is also possible to adopt a Single Input Multiple Output (SIMO) using a plurality of reception antennas 25 with respect to one transmission antenna 23, or a Single Input Single Output (SISO) using a pair of the transmission antenna 23 and the reception antenna 25.

Since the blood pressure measurement device 40 can measure the systolic blood pressure from the waveform of the pulse wave, the above-mentioned algorithm (for detecting the level of the eighth harmonic wave and measuring the systolic blood pressure using the above-mentioned correlation) can be used for any type of measurement device capable of obtaining the pulse wave, regardless of whether it is a measurement device in a contact manner or invasive manner.

Moreover, although the microwave is used as electromagnetic waves in the embodiments, it is also possible to use electromagnetic waves of other frequency bands, such as lasers or visible light, for example.

Furthermore, although the pulse wave is detected from the reflection response of the microwave with which the living body is irradiated and the frequency component decomposition of the pulse wave is executed in the embodiments, it is also possible to measure the systolic blood pressure in consideration of personal characteristic values (age, and the like) in addition to the high-order component electric power of the fundamental frequency of the pulse wave.

### REFERENCE SIGNS LIST

- 1: Experimental Device
- 2: Microwave Circuit
- 3: Control Unit
- 4: Processing Unit
- 5: Blood Pressure Sensor
- 7: Subject
- 21: Oscillator (Osc)
- 22: Transmitter (Tx)
- 23: Transmission Antenna
- 24: Receiver (Rx)
- 25: Reception Antenna
- 26: Mixer (Mix)
- 27: Filter (Filter)
- 31: Pulse Wave
- 33: Component
- 40: Blood Pressure Measurement Device
- 41: CPU
- 42: ROM
- 43: RAM
- 44: Interface
- 45: Input Device
- 46: Output Device
- 47: Storage Device

## Claims

1. A blood pressure measurement device comprising:
a pulse wave obtaining means configured to obtain a pulse wave from a living body;
a frequency component obtaining means configured to obtain a frequency component of the obtained pulse wave; and
a blood pressure measurement means configured to measure a blood pressure of the living body using a frequency component in a band of a predetermined range at a side of a high-frequency range of a fundamental frequency due to the pulse wave, among the obtained frequency components.

2. The blood pressure measurement device according to claim 1, wherein
the blood pressure measurement means adopts a band in which a correlation between the frequency component of the pulse wave and the blood pressure is equal to or greater than a predetermined level as the band of the predetermined range.

3. The blood pressure measurement device according to claim 1 or 2, wherein
the blood pressure measurement means measures the blood pressure using a component of a harmonic of the fundamental frequency in the band of the predetermined range.

4. The blood pressure measurement device according to claim 1, 2, or 3, wherein
the pulse wave obtaining means obtains the pulse wave from a body surface of the living body in a noncontact manner.

5. The blood pressure measurement device according to claim 4, wherein
the pulse wave obtaining means irradiates the living body with an electromagnetic wave having a predetermined frequency and obtains the pulse wave using a reflected wave of the electromagnetic wave.

6. The blood pressure measurement device according to claim 5, further comprising
an informing means configured to inform a content according to a change in the measured blood pressure.

7. A vehicle device comprising the blood pressure measurement device according to claim 6 configured to measure a blood pressure of a driver in a vehicle.

8. The vehicle device according to claim 7, wherein
an antenna for irradiating the driver with the electromagnetic wave and an antenna for receiving the reflected wave of the electromagnetic wave are provided on a backrest portion of a driver's seat.

9. A blood pressure measurement program for causing a computer to realize:
a pulse wave obtaining function for obtaining a pulse wave from a living body;
a frequency component obtaining function for obtaining a frequency component of the obtained pulse wave; and
a blood pressure measurement function for measuring a blood pressure of the living body using a frequency component in a band of a predetermined range at a side of a high-frequency range of a fundamental frequency due to the pulse wave, among the obtained frequency components.
